# EUROPEAN PATENT SPECIFICATION

(11) **EP 0 535 973 B1**
(45) Date of publication and mention of the grant of the patent: **12.03.1997**
(21) Application number: 92309002.1
(22) Date of filing: 02.10.1992
(51) Int. Cl.: A61F 2/46

(54) **An acetabular cup inserter**
Vorrichtung zum Einsetzen einer Hüftpfannenprothese
Dispositif d'insertion de coques acétabulaires

(30) Priority: 04.10.1991 GB 9121132
(43) Date of publication of application: 07.04.1993
(73) Proprietor: JOHNSON & JOHNSON ORTHOPAEDICS, INC., Raynham, Massachusetts 02767-0350 (US)
(72) Inventor: Foley, Frank, Byfleet, Surrey KT14 7AL (GB); Light, Edward, Sway, Lymington (GB)
(74) Representative: Fisher, Adrian John

(56) References cited:
- EP-A- 0 327 509
- EP-A- 0 446 012
- WO-A-82/02145
- WO-A-86/05384
- WO-A-86/05679
- DE-A- 2 852 559
- FR-A- 2 650 174
- FR-A- 2 663 840
- US-A- 2 113 246

## Description

This invention relates to a surgical instrument for inserting prosthetic acetabular cups into a patient's prepared acetabulum.

The procedure for inserting acetabular cups is, at present, normally as follows: First, the cup is mounted onto the forward end of an inserting instrument, which engages the cup and holds it still against relative lateral and rotational motion, and which, in order to do so, cooperates with peripheral regions of the cup. The cup is usually prevented from moving longitudinally with respect to the inserter, and hence falling off, by static frictional forces.

Next, the cup is inserted into the patient's acetabulum, with suitable quantities of bone cement, and is oriented, with the aid of guides on the inserter, correctly. Once oriented, pressure is applied to the cup to embed it in the bone cement, and the inserter is withdrawn.

A second instrument is subsequently introduced, normally into the central socket of the cup, in order for continued pressure to be applied to the cup. It is essential that the cup be kept pressurised for the five or ten minutes during which the bone cement is hardening, in order for the cement to intrude properly into the interstices of the bone surrounding the prepared acetabulum to provide a good join between cup and bone.

A result of the continued pressure, at least during the initial hardening of the cement, is that some excess cement is bound to be extruded from around the periphery of the cup. It is essential that this cement be removed immediately, before it sets hard, so as to leave a clean join between cup and acetabulum and to minimise the quantity of foreign material. Excess material may impair movement, or promote fibrosis or calcification.

Since the inserter engages with peripheral regions of the acetabular cup in order to prevent unwanted movement, the inserter has to be removed to allow access to the cup periphery and to facilitate removal of the excess cement. For this reason, a second, pressurising instrument is required to maintain pressure on the cup.

However, this procedure has a number of disadvantages. One such disadvantage is that removal of one instrument and introduction of another results in a short time during which the bone cement is not pressurised, which is clearly disadvantageous, since the setting process is already under way.

A second disadvantage, with potentially disastrous results, is that the disengagement of the inserter from the cup and the introduction of the pressuriser may dislodge the cup from its ideal orientation at about 45° from the patient's medial plane. As the pressuriser, in order to facilitate removal of excess cement, cannot engage peripheral regions of the cup, it also cannot maintain the cup at a specific orientation relative to itself. Accordingly, the pressuriser cannot be provided with meaningful guides to allow the surgeon to check for misalignments. Once a misalignment has occurred, it is effectively undetectable until symptoms manifest themselves in the patient.

Accordingly, much time and care must be taken by the surgeon during interchange of the surgical instruments to ensure that no such misalignments occur. This is highly inconvenient, even during a routine operation such as hip replacement, especially as it is all performed against the time constraints imposed by the setting cement.

In order to reduce the risk of dislodgement of the cup during removal of the inserter from its frictional engagement therewith, a type of inserter referred to herein as an "ejecting inserter" has been proposed and used.

An example of such an inserter is described in EP-A-67149. The instrument described consists of a ball which fits into the central recess of the acetabular cup and a flange which engages the peripheral region of the cup and maintains the cup at a specific orientation. A drive mechanism is provided to move the ball longitudinally relative to the flange, the ball maintaining its engagement with the inner cavity of the cup. This has the effect of disengaging the flange from the cup while maintaining some pressure, via the ball, on the cup. An inserter such as this reduces the likelihood of dislodgement, but some risk still remains, in addition to which the first disadvantage described above is still present.

WO-A-8605679 discloses an inserter for a prosthetic acetabular cup having a pressure application means and a cup retainer. The cup retainer can be moved relative to the pressure application means whilst the pressure is still applied. Again, the first disadvantage is still present.

The present invention seeks to overcome both of the disadvantages described above by providing an inserter which may also serve as a means of pressurising the inserted cup while excess cement is removed.

Accordingly, the invention provides an instrument for inserting prosthetic acetabular cups, as defined in the claims, including: pressure application means; and a cup retainer capable of being moved relative to the pressure application means between a forward position, in which it is adapted to engage and retain an acetabular cup, and a rearward position; wherein the pressure application means enables pressure to be continually applied to the acetabular cup, irrespective of the position relative thereto of the cup retainer.

The procedure for inserting an acetabular cup is greatly simplified in that the cup is mounted onto the inserter with the cup retainer in its forward position, and inserted into the prepared acetabulum. The cup retainer may then be withdrawn from its forward position, thereby disengaging from the cup, and pulled back to its rearward position, pressure all the while being maintained on the cup. When in its rearward position, the cup retainer does not hinder access to the periphery of the cup, thus allowing removal of excess bone cement.

So long as continued pressure is applied to the cup in this way, the risk of dislodgment or misalignment is effectively negligible, making the surgeon's job that much easier. Also, at no point in the procedure is the cement not pressurised.

Preferably, the instrument includes actuating means operable to move the cup retainer from its forward position to a retracted position in which it is adapted to be out of engagement with the cup.

This provides a convenient way for the surgeon to disengage the cup retainer from the cup whilst minimising his own movements and hence the risk of dislodgement or misalignment.

It is preferred that the retracted position of the cup retainer lies between its forward and rearward positions.

Further to increase the operational convenience of the instrument, it is preferably provided with a hand grip, the actuating means being a trigger or lever mechanism associated with the hand grip.

The surgeon will use the hand grip to stabilise the instrument during insertion, then simply actuate the trigger or lever to release the acetabular cup.

If the hand grip is made integral with the cup retainer, then, following actuation of the trigger or lever, the surgeon need only pull backward on the hand grip to move the cup retainer to its rearward position. If the trigger or lever is mounted on the cup retainer then he need not release the trigger until the cup retainer has been fully withdrawn.

These features are designed to minimise movements during the early part of the procedure, when the cup retainer is in the vicinity of the inserted acetabular cup. It is at this time that movement is potentially most harmful.

In a preferred embodiment of instrument, the trigger or lever mechanism includes a manipulating portion, a pivot and an abutment portion adapted to abut against a receiving part of the instrument. Thus the trigger or lever acts as a "crow-bar" type of lever and a substantial mechanical advantage can be arranged. This is advantageous if, for stability, the cup retainer is retained in its forward position by a relatively large static frictional force or by a clip.

Once the cup retainer has been withdrawn to its rearward position, it is preferable that it may be retained there, in order to allow the surgeon to free his hand. A clip or latch may be provided to this effect.

In a preferred embodiment of the present invention, the means to enable pressure to be applied to the acetabular cup comprises a rigid central rod adapted at its forward end to engage the central recess of the cup, the cup retainer comprising an external sleeve capable of sliding along the rod. Pressure applied to the rearward end of the rod is transmitted through the rod to the forward end, and hence the acetabular cup.

Preferably, to define the forward position of the cup retainer, the central rod includes a stop surface adapted to abut a complementary stop surface on the cup retainer when the cup retainer is in its forward position. A convenient arrangement for achieving this may be obtained with a central rod which comprises a forward portion of relatively large cross-sectional area and a rearward portion of relatively small cross-sectional area, the transition between the two forming the stop surface.

Again, in the preferred embodiment, the cup retainer stop surface consists of a surface of the abutment portion of the trigger or lever mechanism.

In a preferred embodiment of the invention the central rod includes, at its forward end a substantially hemispherical button and, at its rearward end a palm pad. Furthermore, the cup retainer includes, at its forward end a cup-retaining flange which, in turn, includes at least one cup-engaging pin.

The hemispherical button and palm pad may be formed of CELCON (Regd. Trade Mark), or any other biocompatible material. They may be formed of the same material as the remainder of the instrument. Suitable materials are surgical steel, stainless steel, titanium, cobalt or other non-corrosive metals. Any material autoclavable at 138°C for between 3 and 6 minutes is suitable.

The preferred material is stainless steel.

A preferred embodiment of the present invention will now be described with reference to figs. 1-3 of the accompanying drawings, wherein:
fig. 1 is a side view, partly in section, of the instrument with the cup retainer in its rearward position;
fig. 2 is a side view of the instrument with the cup retainer in its forward position;
fig. 3 is a sectional view taken along the line III-III of fig. 1.

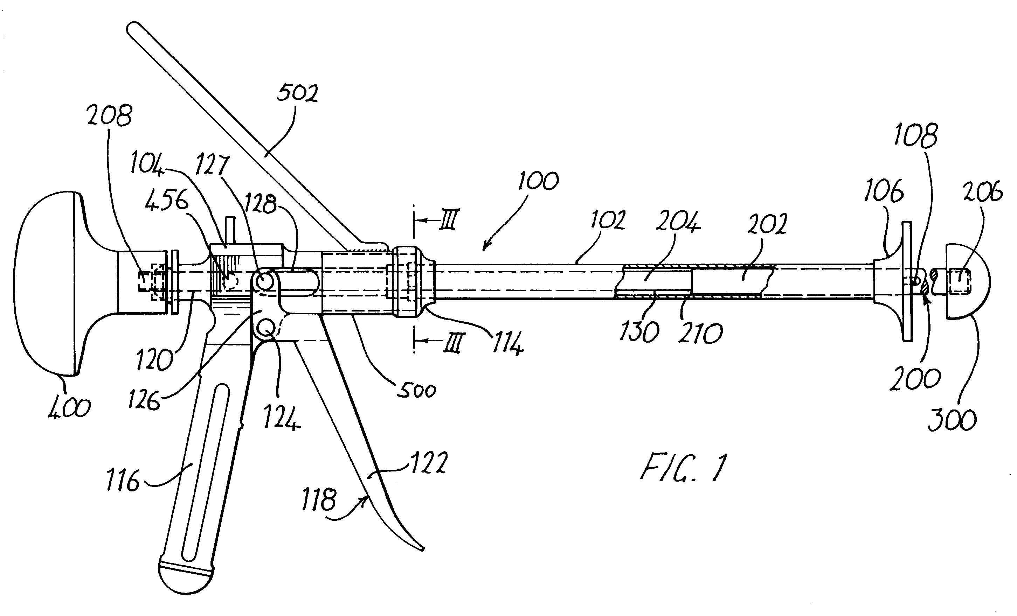

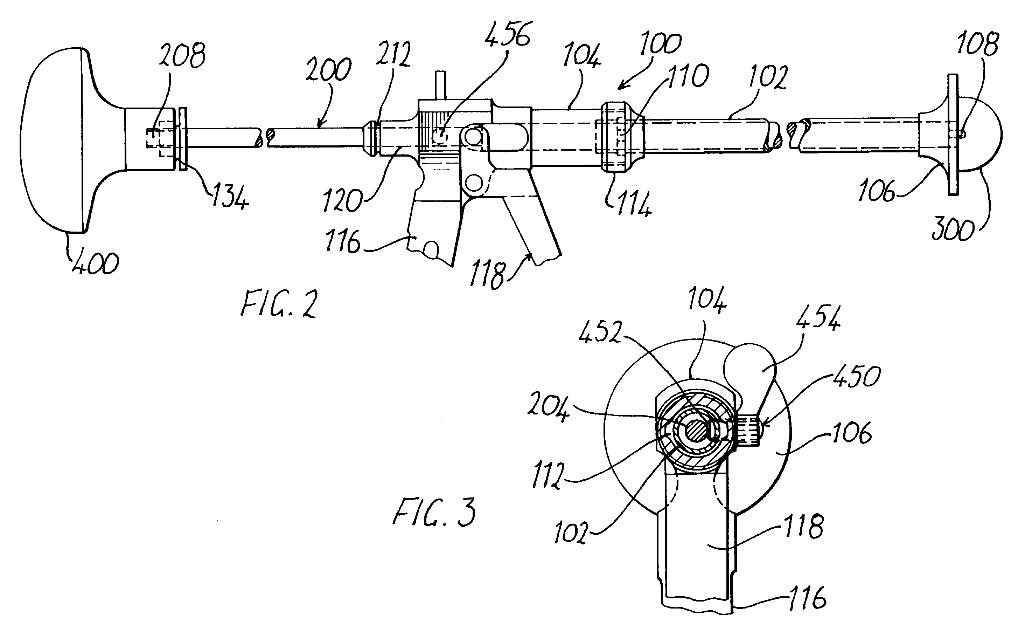

The instrument, as shown in figs. 1-3, includes a cup retainer (100) which consists essentially of two parts, a forward sleeve (102), and a rearward sleeve (104). The forward sleeve (102) has a flange (106) at its forward end, and the flange (106), in turn, carries a pair of cup-engaging pins (108). Towards its rearward end, the forward sleeve (102) is provided with a pair of outstanding locating lugs (110) which fit into complementary locating recesses (fig. 3; 112) at the forward end of the rearward sleeve (104). A screw-threaded collar (114), retained captive on the forward sleeve (102) by the flange (106) and the locating lugs (110), is adapted to engage a complimentary external screw thread on the forward end of the rearward sleeve (104). This enables the forward sleeve (102) to be interchanged with additional sleeves compatible, for example, with cups of different sizes or specifications. The forward sleeve has a central bore (130) extending throughout its length.

The rearward sleeve (104) is also provided with a central bore (120) extending throughout its length. Towards its rearward end, the rearward sleeve (104) is provided with a hand grip (116) and a trigger mechanism (118). The trigger mechanism (118) itself consists of a manipulating portion (122) pivotable relative to the rearward sleeve (104) and hand grip (116) about a pivot axis (124).

Extending upwards from the pivot axis (124) on either side of the rearward sleeve (104) is a connecting plate (126), integrally formed with the manipulating portion (122) of the trigger mechanism (118). Each connecting plate (126) is provided, on its upper inner surface with a boss which consists of the inward projection of a cylindrical pin (127), the function of which will be discussed in detail below. An opening (128) is provided in each side of the rearward sleeve (104) to accommodate its respective boss during the full range of movement of the trigger mechanism (118).

The central bore (120) of the rearward sleeve is narrower rearward of the openings (128) than it is forward thereof. The diameter forward of the openings is the same as that of the central bore (130) of the forward cylinder (102).

The instrument further includes a central rod (200) which consists essentially of two parts, a forward rod (202) and a rearward rod (204). Both the forward end of the forward rod (202) and the rearward end of the rearward rod (204) are provided with screw threads (206; 208), the former for attachment of a hemispherical button (300), the latter a palm pad (400). The forward rod (202) has a larger diameter than the rearward rod (204) and their respective diameters correspond with those of the bore of the forward sleeve (102) and of the bore of the rearward sleeve (104), rearward of the openings (128) therein. By "corresponding" is meant not so nearly matched as to form an interference fit, but sufficiently close to enable static frictional forces to have an appreciable effect. A circumferential step (210) obtains at the junction between the two parts (202, 204).

In its assembled form, the central rod (200) is inserted into the cup retainer (100) from the front. When the central rod (200), is fully inserted into the cup retainer (100), the rearward rod (204) extends rearwardly of the cup retainer, as shown in fig. 2. The step (210) between the rearward (204) and forward (202) rods forms a receiving part against which an abutment portion, that is to say the pair of inwardly extending bosses, of the trigger mechanism (118) abuts. The forward end of the forward rod (202) lies flush with the front face of the flange (106) on the cup retainer (100).

Rearward movement of the manipulating portion (122) of the trigger mechanism (118) causes the upper regions of the connecting plates (126), and hence the bosses, to advance, advancing the central rod (200) with respect to the cup retainer (100) by virtue of the abutment of the bosses against the circumferential step (210). The entire cup retainer (100) may then be slid backwards along the rearward rod (204).

When the cup retainer is fully withdrawn, a spring clip (134) on the palm pad (400) engages with a circumferential groove (212) on the rear of the cup retainer (100) to retain the cup retainer in its rearward position. The action of the spring clip (134) can be overcome by a sharp tap on the button (300) at the forward end of the central rod (200).

Figs. 1 and 2 show preferred shapes for the hemispherical button (300) and the palm pad (400), both being circular in lateral cross-section.

An alignment guide is shown in fig. 1 and consists of a guide sleeve (500) and an alignment rod (502) extending angularly therefrom, preferably at about 45°. The guide sleeve (500) is adapted frictionally to engage the narrowed portion of the rearward sleeve (104), which can be seen between the captive collar (114) and the openings (128) in fig. 2. For the surgeon's convenience, the guide (500, 502) may be rotated relative to the cup retainer (100), if desired, but fictionally engages therewith to prevent accidental rotation.

As can be seen from fig. 3, in order to prevent relative rotation of the cup retainer (100) and the central rod (200) during insertion of an acetabular cup, there is provided a friction screw lock (450). This screw lock consists of a brass screw (452) which, in its inserted position, clamps the rearward rod (204), and a thumb lever (454). The screw is received in a threaded hole (456) which can be seen in figs. 1 and 2.

It will, of course, be appreciated that the present invention has been described above purely by way of example, and that modifications of detail may be made without departing from its scope as defined by the claims.

## Claims

1. An instrument for inserting prosthetic acetabular cups including:
pressure application means wherein the pressure application means enables pressure to be continually applied to the acetabular cup, irrespective of the position relative thereto of a cup retainer (100);
the cup retainer (100) capable of being moved relative to the pressure application means between a forward position, in which it is adapted to engage and retain an acetabular cup and a rearward position;
actuating means, integral with the cup retainer (100), operable to move the cup retainer (100) from its forward position to a retracted position in which it is adapted to be out of engagement with the cup; and a hand grip (116);
characterised in that the actuating means comprises a trigger or lever mechanism (118) associated with the hand grip (116);
wherein the trigger or lever mechanism (118) includes a manipulating portion (122), a pivot axis (124) and an abutment portion, and the pressure application means comprises a rigid central rod (200) adapted at its forward end to engage a central recess of the cup, the cup retainer (100) comprising an external sleeve capable of sliding along the rod (200), the rod (200) including a stop surface adapted to abut a complementary stop surface on the abutment portion of the trigger or lever mechanism (118) when the cup retainer (100) is in its forward position.

2. An instrument according to claim 1 in which the retracted position of the cup retainer (100) lies between its forward and rearward positions.

3. An instrument according to claim 1 or 2 in which the hand grip (116) is integral with the cup retainer (100).

4. An instrument according to any one of the preceding claims further including means for retaining the cup retainer (100) in its rearward position.

5. An instrument according to claim 4 in which the means for retaining the cup retainer (100) in its rearward position comprises a clip or latch (134).

6. An instrument according to any one of the preceding claims, in which the central rod (200) comprises a forward portion of relatively large cross-sectional area and a rearward portion of relatively small cross-sectional area, the transition between the two forming the stop surface.

7. An instrument according to any one of the preceding claims in which the central rod (200) includes, at its forward end a substantially hemispherical button (300) and, at it is rearward end a palm pad (400).

8. An instrument according to any one of the preceding claims in which the cup retainer (100) includes at its forward end a cup retaining flange (106).

9. An instrument according to claim 8 in which the flange (106) includes at least one cup-engaging pin (108).

## Patentansprüche

1. Instrument zum Einsetzen einer künstlichen Hüftgelenkspfannenprothese, mit
einer Druckaufbringungseinrichtung, wobei es die Druckaufbringungseinrichtung ermöglicht, auf die Hüftgelenkspfannenprothese unabhängig von der Position relativ zu einem Pfannenhalter (100) kontinuierlich Druck auszuüben;
wobei der Pfannenhalter (100) relativ zu der Druckaufbringungseinrichtung zwischen einer vorderen Stellung, in der er mit einer Hüftgelenkspfannenprothese in Eingriff kommen und diese festhalten kann, und einer hinteren Stellung bewegbar ist;
einer mit dem Pfannenhalter (100) ein Ganzes bildenden Betätigungseinrichtung, die so betätigt werden kann, daß sich der Pfannenhalter (100) von seiner vorderen Stellung in eine zurückgezogene Stellung bewegt, in der er aus dem Eingriff mit der Pfanne kommen kann; und mit einem Handgriff (116);
dadurch **gekennzeichnet**, daß die Betätigungseinrichtung einen Auslöse- oder Hebelmechanismus (118) umfaßt, der dem Handgriff (116) zugeordnet ist;
wobei der Auslöse- oder Hebelmechanismus (118) einen Manipulationsabschnitt (122), eine Schwenkachse (124) und einen Stoßabschnitt umfaßt und die Druckaufbringungseinrichtung einen starren zentralen Stab (200), der dafür vorgesehen ist, an seinem vorderen Ende mit einer zentralen Vertiefung in der Pfanne in Eingriff zu kommen, wobei der Pfannenhalter (100) eine äußere Hülse aufweist, die entlang des Stabes (200) gleiten kann, und wobei der Stab (200) eine Anschlagfläche besitzt, die dafür vorgesehen ist, an eine komplementäre Anschlagfläche am Stoßabschnitt des Auslöse- oder Hebelmechanismusses (118) anzustoßen, wenn sich der Pfannenhalter (100) in seiner vorderen Stellung befindet.

2. Instrument nach Anspruch 1, wobei die zurückgezogene Stellung des Pfannenhalters (100) zwischen seiner vorderen und seiner hinteren Stellung liegt.

3. Instrument nach Anspruch 1 oder 2, wobei der Handgriff (116) mit dem Pfannenhalter (100) ein Ganzes bildet.

4. Instrument nach einem der vorstehenden Ansprüche, mit einer Einrichtung zum Halten des Pfannenhalters (100) in seiner hinteren Stellung.

5. Instrument nach Anspruch 4, wobei die Einrichtung zum Halten des Pfannenhalters (100) in seiner hinteren Stellung eine Klammer oder eine Rastvorrichtung (134) umfaßt.

6. Instrument nach einem der vorstehenden Ansprüche, wobei der zentrale Stab (200) einen vorderen Abschnitt mit einer relativ großen Querschnittsfläche und einen hinteren Abschnitt mit einer relativ kleinen Querschnittsfläche aufweist, wobei der Übergang dazwischen die Anschlagfläche bildet.

7. Instrument nach einem der vorstehenden Ansprüche, wobei der zentrale Stab (200) an seinem vorderen Ende einen im wesentlichen halbkugelförmigen Knopf (300) und an seinem hinteren Ende eine Handballenauflage (400) aufweist.

8. Instrument nach einem der vorstehenden Ansprüche, wobei der Pfannenhalter (100) an seinem vorderen Ende einen Pfannenhalteflansch (106) aufweist.

9. Instrument nach Anspruch 8, wobei der Flansch (106) wenigstens einen mit der Pfanne in Eingriff kommenden Stift (108) aufweist.

## Revendications

1. Instrument pour l'insertion de coques acétabulaires prothétiques comprenant :
un moyen d'application de pression permettant d'exercer une pression continue sur la coque acétabulaire, quelle que soit la position par rapport à cette coque d'un dispositif de retenue de coque (100);
le dispositif de retenue de coque (100) pouvant être déplacé par rapport au moyen d'application de pression entre une position avant, dans laquelle il est à même d'engager et de retenir une coque acétabulaire, et une position arrière;
un moyen d'actionnement, venu d'une seule pièce avec le dispositif de retenue de coque (100), à même d'être actionné pour déplacer le dispositif de retenue de coque (100) de sa position avant vers une position rétractée dans laquelle il peut être désengagé de la coque, et une poignée (116),
caractérisé en ce que le moyen d'actionnement comprend un mécanisme à détente ou à levier (118) associé à la poignée (116);
dans lequel le mécanisme à détente ou à levier (118) comprend une partie de manipulation (122), un axe de pivotement (124) et une partie de butée, et le moyen d'application de pression comprend une tige centrale rigide (200) à même d'engager à son extrémité avant une cavité centrale de la coque, le dispositif de retenue de coque (100) comprenant une gaine externe pouvant coulisser le long de la tige (200), la tige (200) comprenant une surface d'arrêt propre à venir en butée contre une surface d'arrêt complémentaire sur la partie de butée du mécanisme à détente ou à levier (118) lorsque le dispositif de retenue de coque (100) se trouve dans sa position avant.

2. Instrument suivant la revendication 1, dans lequel la position rétractée du dispositif de retenue de coque (100) se trouve entre ses positions avant et arrière.

3. Instrument suivant la revendication 1 ou 2, dans lequel la poignée (116) est venue d'une seule pièce avec le dispositif de retenue de coque (100).

4. Instrument suivant l'une quelconque des revendications précédentes comprenant en outre un moyen permettant de retenir le dispositif de retenue de coque (100) dans sa position arrière.

5. Instrument suivant la revendication 4, dans lequel le moyen permettant de retenir le dispositif de retenue de coque (100) dans sa position arrière comprend une attache ou un verrouillage (134).

6. Instrument suivant l'une quelconque des revendications précédentes, dans lequel la tige centrale (200) comprend une partie avant de section transversale relativement grande et une partie arrière de section transversale relativement petite, la transition entre les deux formant la surface d'arrêt.

7. Instrument suivant l'une quelconque des revendications précédentes, dans lequel la tige centrale (200) comprend, à son extrémité avant, un bouton sensiblement hémisphérique (300) et, à son extrémité arrière, un coussinet de paume (400).

8. Instrument suivant l'une quelconque des revendications précédentes, dans lequel le dispositif de retenue de coque (100) comprend, à son extrémité avant, une collerette de retenue de coque (106).

9. Instrument suivant la revendication 8, dans lequel la collerette (106) comprend au moins une tenon d'engagement de coque (108).
